(19) Europäisches Patentamt
European Patent Office
Office européen des brevets

(11) EP 2 019 666 B1

(12) EUROPEAN PATENT SPECIFICATION

(45) Date of publication and mention of the grant of the patent:
**12.03.2014 Bulletin 2014/11**

(21) Application number: **07734379.6**

(22) Date of filing: **24.04.2007**

(51) Int Cl.:
*A61K 47/10* (2006.01) *A61K 47/14* (2006.01)
*A61K 47/34* (2006.01) *A61K 47/44* (2006.01)
*A61K 31/704* (2006.01) *A61K 31/192* (2006.01)
*A61P 29/00* (2006.01) *A61K 9/06* (2006.01)
*A61K 9/00* (2006.01)

(86) International application number:
**PCT/IB2007/001061**

(87) International publication number:
**WO 2007/129162 (15.11.2007 Gazette 2007/46)**

(54) **Pharmaceutical preparations for transdermal use**

Pharmazeutische Zubereitungen zur transdermalen Anwendung

Préparations pharmaceutiques à usage transdermique

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **26.04.2006 IT BO20060313**

(43) Date of publication of application:
**04.02.2009 Bulletin 2009/06**

(73) Proprietor: **Alma Mater Studiorum -Universita' di Bologna**
**40126 Bologna (IT)**

(72) Inventors:
- **CAVALLARI, Cristina**
  **40138 BOLOGNA (IT)**
- **LUPPI, Barbara**
  **40055 Villanova Di Castenaso (IT)**
- **DI PIETRA, Anna Maria**
  **40139 BOLOGNA, Italy (IT)**
- **RODRIGUEZ, Lorenzo**
  **40069 ZOLA PREDOSA (IT)**

(74) Representative: **Mangini, Simone et al**
**Studio Torta S.p.A.**
**Via Viotti, 9**
**10121 Torino (IT)**

(56) References cited:
**EP-A- 1 457 202**
**WO-A-96/41635**
**WO-A-2004/100866**
**FR-A- 2 725 134**
**US-B1- 6 399 093**

- **PIFFERI G: "COMPATIBILITA CHIMICO-FI-SICA TRA TIOCOLCHICOSIDE E FARMACI ANTINFIAMMATORI NON STEROIDEI. - Chemico-physical compatibility of thio-colchicoside with nonsteroidic anti-inflammatory drugs", BOLLETTINO CHIMICO FARMACEUTICO, SOCIETA EDITORIALE FARMACEUTICA, MILANO, IT, vol. 132, no. 6, 1 June 1993 (1993-06-01), pages 203-209, XP000566124, ISSN: 0006-6648**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 019 666 B1

## Description

### TECHNICAL FIELD

The present invention relates to a formulation for transdermal use comprising an emulgel, thiocolchicoside and a substantially lipophilic compound, having a pharmacological activity, which is selected from the group consisting of: ibuprofen, ketoprofen, naproxen and sulindac.

Furthermore, the present invention relates to a formulation for transdermal use comprising thiocolchicoside, ibuprofen and a pharmaceutically acceptable vehicle suitable for transdermal use.

The presently claimed formulations are used as pharmaceuticals in the treatment of pain and/or inflammation.

### BACKGROUND ART

Usually, in most cases, the medicines are administered by the oral route or by the parenteral or the transdermal ones.

The parenteral administration of medicines can involve the contamination of internal tissues with extraneous substances or bacteria.

The administration of many medicines by oral route is sometime not possible or not desirable since some medicines can decompose, be insufficiently absorbed or damaged in the gastrointestinal tract. Besides, the maintenance of constant and optimal hematic concentrations of the medicines orally administered is often hardly obtainable.

The transdermal administration of medicines can be used to get a local as well as a systemic action; the systemic action is determined by the absorption in the blood circulation of the medicines.

Although the topical application of medicines does not involve all the drawbacks of the oral and parenteral administrations, relatively few medicines are administered by the transdermal route. This is mainly due to the fact that the skin has an impermeability which does not allow many medicines to reach the sub-cutaneous level in sufficient quantity.

From the above mentioned arguments it can be also inferred that there is still a notable need to make available new formulations for transdermal use, that can overcome, at least partly, the above mentioned drawbacks, that are easy and cheap to implement and that allow the penetration to the sub-cutaneous level of medicines relatively poorly cable of permeating into the horny layer.

US6399093 discloses a combination of colchicine and etodolac in a topical gel composition. The transdermal combination of colchicine and ibuprofen is also disclosed, but not in the form of a topical gel. The topical combinations are used for the treatment of inflammation and pain.

WO96/41635 discloses a combination of thiocolchicoside and diclofenac in the form of a topical gel. The composition is used for the treatment of inflammation and pain.

FR 2 725 134 discloses an oral administration of thiocolchicoside and ibuprofen.

PIFFERI G: "COMPATIBILITA CHIMICO-FI SICA TRA TIOCOLCHICOSIDE E FARMACI ANTINFIAMMATORI NON STEROIDEI. - Chemical-physical compatibility of thiocolchicoside with nonsteroidic anti-inflammatory drugs", BOLLETTINO CHIMICO FARMACEUTICO, SOCIETA EDITORIALE FARMACEUTICA, MILANO, IT, vol. 132, no. 6, 1 June 1993 (1993-06-01), pages 203-209, discloses an injectable formulation comprising thiocolchicoside and ketoprofene.

### DISCLOSURE OF INVENTION

The aim of the present invention is to provide formulations for the transdermal administration and for use in therapy.

Unless the contrary is not expressly specified, in the present text the following terms are introduced with the following meaning.

For "pharmaceutically acceptable vehicle" it is intended a composition of one or more substances (excipients, diluents etc.) suitable to be administered to humans and to mammals. Particularly, the substances included in the pharmaceutically acceptable vehicle have to be suitable to be mixed with the active principles contained in the formulation so as to avoid reduction of the effectiveness of such active principles; the substances included in the vehicle have also to be sufficiently pure and their toxicity has to be sufficiently low to allow the administration to humans and to mammals. Examples of the substances contained in the vehicle are: ethylene glycol, propylene glycol, glycerol, liquid paraffin, ethanol, water, antioxidants, preservatives, emulsifiers, gelling materials.

In the present text for "emulgel" it is intended a composition with a colloidal structure and comprising a tenside agent. In particular, compositions, containing an oily phase, a aqueous phase, a tenside (or emulsifier) and a gelling agent, which has the function to gel the aqueous phase, are considered "emulgels". The tenside has the function to emulsify the oily phase in the aqueous phase. Preferably, the gelling agent contains a Sepigel, particularly the Sepigel 305 (SEPPIC).

Sepigel, 305 has both a gelling and an emulsifying activity and comprises: polyacrylamide and C13-14 isoparaffin and Laureth-7 (lauric alcohol 7 ethosilated).
In the present text for "substantially lipophilic component" it is intended a compound that can be dissolved more easily in a lipidic phase (in particular, liquid paraffin) than in a aqueous one (in particular, water).
In the present text "LogP" means logaritm in base ten of the octanol/water partition coefficient of a component. The LogP is defined and calculated as described in the article of La Rotonda, M. I., Amato, G. , Barbato, F. , Silipo, C., Vittoria, A. , Quant. Struct. Act., Relat. (1983) 2, 168, whose content is herein included for completeness of description.
In the present text for "pharmaceutically acceptable salt" of a compound, it is intended a coordination of the compound with a further chemical derivative generating in solution two ions of opposite sign, even not tied up among them.
Examples of methods for the preparation of such salts include the followings: addition of inorganic acids (for instance hydrochloric acid, hydrobromic acid, sulphuric acid, phosphoric acid) or organic acids (for instance acetic acid, oxalic acid, maleic acid, mehanesulphonic acid, salicylic acid, succinic acid, citric acid) to the compound; substitution of an acidic proton of the compound with a metallic cation (for instance a cation of an alkaline metal or aluminum); transfer of a acidic proton of the compound to an organic base (for instance dimethylammine, triethylammine) and coordination with such organic base. The compounds (thiocolchicoside, substantially lipophilic derivatives, ibuprofen) contained in the formulations object of the present invention must be intended, unless the contrary is specified, as molecules in their neutral form and/or as ions of pharmaceutically acceptable salts.
In the present text "h" means hours (time).

BRIEF DESCRIPTION OF THE DRAWING

A number of embodiments of the present invention will be described by way of example with reference to the accompanying drawings, in which:

- figure 1 schematically illustrate a cell of Franz used to carry out in vitro tests of transdermal permeation.

BEST MODE FOR CARRYING OUT THE INVENTION

In accordance with a first aspect of the present invention, there is provided a formulation for transdermal use comprising an emulgel, thiocolchicoside and a substantially lipophilic compound,having a pharmacological activity, which is selected from the group consisting of: ibuprofen, ketoprofen, naproxen and sulindac.
It is important to point out, that it can be experimentally observed that, surprisingly, the action of thiocolchicoside and of the emulgel substantially improves the penetration to sub-cutaneous level of the substantially lipophilic compounds.
The substantially lipophilic compound has a LogP value: between 1.5 and 4.5, between 1.5 and 3.8, between 3.1 and 3.7, between 3.4 and 3.6.
According to increasingly preferred embodiments, the emulgel comprises an oil or a mixture of oils, in which the w/w solubility of the lipophilic compound is between 5% and 20%, between 8.2% and 14.5%, and approximately between 8.3% and approximately 11.5%.
According to some embodiments, the substantially lipophilic compound is selected in the group of: ibuprofen (LogP = 3.5), ketoprofene (LogP = 3.12), naproxen (LogP = 3.34), sulindac (LogP = 3.42).
Preferably, the substantially lipophilic compound is ibuprofen.
According to increasingly preferred embodiments, the w/w (weight/weight) concentration of the lipophilic compound is between about 2% and about 16%, between about 3% and about 15%, between about 4% and about 12%, between about 9% and about 11%, of about 10%.
In accordance with a further aspect of the present invention, there is provided a formulation for transdermal use comprising thiocolchicoside, ibuprofen and a pharmaceutically acceptable vehicle suitable for transdermal use.
It is important to point out that we have experimentally observed that the actions of ibuprofen and thiocolchicoside surprisingly combine so as to improve the sub-cutaneous penetration.
According to preferred embodiments, the pharmaceutically acceptable vehicle comprises an emulgel; the emulgel is defined, more preferably, as above.
Preferably, the formulations as above mentioned comprise at least an absorption enhancer. According to preferred embodiments, the formulations as above defined comprise one or more of the following derivatives: urea, ethanol, Escine, diethylammine and dimethyaminoethanol. Preferably, the urea is at a w/w concentration between about 5% and about 40%; more preferably, the w/w concentration of urea is between about 5% and about 20%. Preferably, ethanol is at a w/w concentration between about 5% and about 20%. Preferably, Escine is at a w/w concentration between about 0.5 and about 2%, more preferably at about 1%. Preferably, diethylammine is at a w/w concentration between about 0.5 and about 2%, more preferably around 1%. Preferably, dimethylaminoethanol is introduced at a w/w concentration between about 0.5 and 2%, more preferably at about 1%.

In the formulations as above defined, the derivative of colchicine is thiocolchicoside:

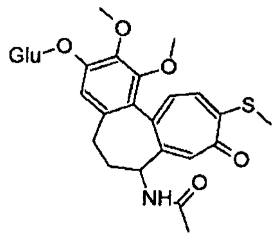

According to some formulations, the emulgel comprises one or more of the following component: polyacrylamide, C13-C14 isoparaffin and Laureth-7.

According to some embodiments, the emulgel comprises one or more of the following components: Sepigel 305, Mygliol 812, Cremophor RH40, water and propilenic glycol.

Preferably, the formulation has a w/w percentage of Sepigel 305 between 2% and 11%, more preferably between 2.5% and 3%. Preferably, the formulation has a w/w percentage of water between 44% and 65%. Preferably, the formulation has a w/w percentage of propylene glycol (acting as humectant) of about 5%. Preferably, the formulation has a w/w percentage of Mygliol 812 between about 5% and about 13%, more preferably between about 6% and about 12%, even more preferably, between about 11.7% and about 6.3%. Preferably, the formulation has a w/w percentage of Cremophor RH40 between about 5% and about 8%, more preferably between about 6% and about 12%, even more preferably, of about 6.3%.

According to preferred embodiments, in the formulations as above mentioned, the concentration thiocolchicoside is between about 0.03% and about 0.25%.

According to increasingly preferred embodiments, the formulation has a w/w concentration of ibuprofen: between about 2% and about 16%, between about 3% and about 15%, between about 4% and about 12%, between about 9% and about 11%, of about 10%.

In accordance with a further aspect of the present invention, there are provided the formulations as above defined for use as pharmaceuticals; preferably, for use in the treatment of pain and inflammation.

In accordance with a further aspect of the present invention, there are provided uses of the formulations as above defined for the preparation of anti-inflammatory pharmaceutical products; particularly, for the preparation of pharmaceutical products to be administered to mammals for treating pain and inflammation.

In accordance with a further aspect of the present invention, there are provided formulations as above defined for use in the treatment of pain and inflammation, wherein the formulation is administered topically to mammals.

Further characteristics of the present invention will result from the description of the following examples:

**Example 1**

Tests have been carried on for verifying the solubility of ibuprofen in different oils; the results of these tests are resumed in Table 1

Table 1

| OILS | almonds | Paraffin | paraffin Cremoph | Isopropyl miristato | Isopropyl Cremophor | Mygliol 812 | Mygliol 812 Cremophor |
|---|---|---|---|---|---|---|---|
| [ ] p/p | 1.9 | 2.0 | 2.7 | 6.5 | 8.3 | 10.7 | 11.5 |

In the first line are reported the types of oils or mixtures of used oils. Solubilities are reported as w/w percentages (solute/solvent).

Mygliol 812 (SASOL) is a synthetic triglyceride containing 75% octanoic acid (caprylic acid) and 25% of decanoic acid (capric acid).

Cremophor means Cremophor RH 40 (BASF), which is a solubility improving agent and an emulsifier obtained through a reaction between 45 moles of ethylene oxide and a mole of hydrogenated castor oil.

**Example 2**

The present example describes alternatives embodiments of the formulation and a methodology to obtain it.

A container of 50 mL containing liquid paraffin and Cremophor RH (ethoxilated hydrogenated castor oil) exactly weighed was placed in a thermostated bath. To the stirred oily mixture, ibuprofen was added in small portions carefully avoiding to exceed the temperature of 60°C at which degradation of the active principle begins. The obtained oily phase was left in the thermostat during the preparation of the aqueous phase.

The aqueous phase was prepared pouring in a mortar the gelling agent (Sepigel 305) and adding to it a solution of thiocolchicoside in a mixture of water and propylene glycol (humectant). The solution was added in small amounts under continuous slow mixing.

The final formulation was then obtained adding the oily phase to the aqueous one. The formulation was then set inside a tube and stored in refrigerator.

| FORMULATION 1 | |
|---|---|
| Ibuprofen | 10.00% |
| Thiocolchicoside | 0.05% |
| Sepigel 305 | 2.70% |
| Cremophor RH40 | 6.30% |
| paraffin oil | 11.70% |
| propylene glycol | 5.00% |
| Water | 64.25% |

| FORMULATION 2 | |
|---|---|
| Ibuprofen | 10.00% |
| Thiocolchicoside | 0.05% |
| Sepigel 305 | 2.70% |
| Cremophor RH40 | 6.30% |
| Paraffin oil | 11.70% |
| Propylene glycol | 5.00% |
| Water | 59.25% |
| Urea | 5.00% |

| FORMULATION 3 | |
|---|---|
| Ibuprofen | 10.00% |
| Thiocolchicoside | 0.05% |
| Sepigel 305 | 2.70% |
| Cremophor RH40 | 6.30% |
| Isopropil myristato | 11.70% |
| Propilene glycol | 5.00% |
| Water | 59.25% |
| Urea | 5.00% |

| FORMULATION 4 | |
|---|---|
| Ibuprofen | 10.00% |

(continued)

| FORMULATION 4 | |
|---|---|
| Thiocolchicoside | 0.05% |
| Sepigel 305 | 2.70% |
| Cremophor RH40 | 6.30% |
| Mygliol 812 | 11.70% |
| Propilene Glycol | 5.00% |
| Water | 44.25% |
| Urea | 20.00% |

According to the formulations 2 to 4, it must be pointed out that urea is a moisturizing agent, that favours to loosen the intermolecular bonds keeping together the protein structures of the epidermis; as consequence, a softening of the horny layer and an improvement of permeability of the drugs contained in the formulation is achieved. The urea can be included up to 40% in the pharmaceutical formulations.

| FORMULATION 5 | |
|---|---|
| Ibuprofen | 10.00% |
| Thiocolchicoside | 0.05% |
| Sepigel 305 | 2.70% |
| Cremophor RH40 | 6.30% |
| Paraffin oil | 6.70% |
| Oleic acid | 5.00% |
| Propylene glycol | 5.00% |
| Water | 64.25% |

Oleic acid increases the fluidity altering the regular structure of the horny layer, reducing in this way its barrier function.

| FORMULATION 6 | |
|---|---|
| Ibuprofen | 10.00% |
| Thiocolchicoside | 0.05% |
| Sepigel 305 | 2.70% |
| Cremophor RH40 | 6.30% |
| Mygliol 812 | 6.70% |
| Ethanol | 5.00% |
| Propilene glycol | 5.00% |
| Water | 64.25% |

| FORMULATION 7 | |
|---|---|
| Ibuprofen | 10.00% |
| Thiocolchicoside | 0.05% |
| Sepigel 305 | 2.70% |

(continued)

| FORMULATION 7 | |
|---|---|
| Cremophor RH40 | 6.30% |
| Mygliol 812 | 6.70% |
| Ethanol | 20.00% |
| Propilene glycol | 5.00% |
| Water | 49.25% |

Relatively to the formulations 6 and 7, it must be pointed out that the ethanol increases the solubility of the active principle (ibuprofen) in the biological membranes and in the vehicle, it penetrates in the horny layer and it has the ability to reduce the barrier function of the horny layer extracting the lipids contained therein.

| FORMULATION 8 | |
|---|---|
| Ibuprofen | 10.00% |
| Thiocolchicoside | 0.05% |
| Sepigel 305 | 2.70% |
| Cremophor RH40 | 6.30% |
| Mygliol 812 | 11.70% |
| Escine | 1.00% |
| Propilene glicol | 5.00% |
| Water | 63.25% |

It can be pointed out that the Escine increases the venous tonicity and its evident strong anti-inflammatory activity; it acts in the initial phase of the inflammation antagonizing the formation of the edema, it normalizes the permeability of the vascular wall altered by the inflammation. Moreover, escine has an analgesic and antiflogistic activity.

| FORMULATION 9 | |
|---|---|
| Ibuprofen | 10.00% |
| Thiocolchicoside | 0.05% |
| Sepigel 305 | 2.70% |
| Cremophor RH40 | 6.30% |
| Mygliol 812 | 11.70% |
| DEA | 1.00% |
| Propilene glycol | 5.00% |
| Water | 63.25% |

DEA means diethylammine, that increases the solubility of ibuprofen in the vehicle and permeation through thereof through the horny layer.

| Formulation 10 | |
|---|---|
| Ibuprofen | 10.00% |
| Thiocolchicoside | 0.05% |
| Sepigel 305 | 2.70% |

(continued)

| Formulation 10 | |
|---|---|
| Cremophor RH40 | 6.30% |
| Mygliol 812 | 11.70% |
| DMEA | 1.00% |
| Propilene glycol | 5.00% |
| Water | 63.25% |

DMEA means dimetylamminoethanol that increases the solubility of ibuprofen in the vehicle and the permeation speed thereof through the horny layer. Although the biological role of the DMEA has not been clarified in univocal way yet, several studies seem to point out that it is a precursor of the neurotransmitter acethylcholine (Ach), that assists the control of the more elevated cognitive mechanisms and of all the motor functions.

| Formulation 11 | |
|---|---|
| Ibuprofen | 10.00% |
| Thiocolchicoside | 0.05% |
| Sepigel 305 | 4.00% |
| Cremophor RH40 | 6.30% |
| Mygliol 812 | 11.70% |
| Escine | 1.00% |
| Propilene glycol | 5.00% |
| Water | 60.95% |
| DEA | 1.00% |

| Formulation 12 | |
|---|---|
| Ibuprofen | 10.00% |
| Thiocolchicoside | 0.05% |
| Sepigel 305 | 4.00% |
| Cremophor RH40 | 6.30% |
| Mygliol 812 | 11.70% |
| Escine | 1.00% |
| Propilene glycol | 5.00% |
| Water | 60.95% |
| DMEA | 1.00% |

| Formulation 13 | |
|---|---|
| Ibuprofen | 10.00% |
| Thiocolchicoside | 0.05% |
| Sepigel 305 | 10.00% |
| Cremophor RH40 | 6.30% |

(continued)

| Formulation 13 | |
|---|---|
| Mygliol 812 | 11.70% |
| Escina | 1.00% |
| Propilene glycol | 5.00% |
| Water | 54.95% |
| DMEA | 1.00% |

| Formulation 14 | |
|---|---|
| Ibuprofen | 10.00% |
| Thiocolchicoside | 0.05% |
| Sepigel 305 | 2.70% |
| Cremophor RH40 | 6.30% |
| Isopropyl myristate | 11.70% |
| Propilene glycol | 5.00% |
| Water | 64.25% |

| Formulation 15 | |
|---|---|
| Ibuprofen | 10.00% |
| Thiocolchicoside | 0.05% |
| Sepigel 305 | 2.70% |
| Cremophor RH40 | 6.30% |
| Mygliol 812 | 11.70% |
| Propilene glycol | 5.00% |
| Water | 64.25% |

**Example 3**

The present example describes *in vitro* tests of the permeation of thiocolchicoside and ibuprofen through horny layer.

In figure 1 a cell of Franz is shown. Like most of the release cells, it is made of two compartments of glass: a donor compartment 2 with the shape of a cylinder, containing the semisolid donor phase and an acceptor compartment 3, containing the receiving liquid phase. A sampling port 4 is connected to the bottom of compartment 3. A membrane 5 of separation between the formulation and the liquid acceptor separates the two compartments. Several elastic strings 6 keep the acceptor 2 and donor 3 compartments in contact. A second port can also be included so as to obtain a flow cell.

The permeation tests were made using cell 1 and, as membrane 5, the skin obtained from the ear of a pig.

Receiving compartment 3, wherein a magnetic bar has been introduced, of cell 1 has previously been filled with 100 mLs of a phosphate buffer solution at pH 7.4. On the surface of the donor compartment 2 a skin barrier was applied, uniformly distributing on it 1 g of formulation exactly weighed.

The system, closed at the top with a transparent film, was placed inside a thermostatic bath for 6 hours at 37°C and continuously stirred.

At regular intervals of one h, a sample of 1 mL has been withdrawn through port 4 of cells; the same volume, 1 mL, of the receiving phase, buffer phosphate pH 7.4, was added to the cell with a graduated pipette.

The collected samples have been analysed by means of HPLC. Chromatographic analyses were performed,

at environmental temperature, using a column Phenomenex Luna C18 (250x4.60 mms I.D., 5. μm). The separations were achieved at isochratic conditions employing a mobile phase made of a mixture of phosphate buffer at pH 7 (0.025 Ms) - acetonitrile-methanol (50:25:25 v/v/vs) at flow speed of of 1 mL/min. The UV detector was set at the following wavelengths: 224 nm and 260 nm, for ibuprofen and thiocolchicoside, respectively. The calibrating curves of thiocolchicoside and ibuprofen were achieved reporting into a graph the areas of the operating peaks as a function of the corresponding concentrations of the analysed compound.

The standard solutions of ibuprofen within the interval of linearity (0.0036-0.0144 mg/mL) were prepared in methanol-phosphate buffer at pH 4.5 (90:10 v/vs) and then injected in triplicate; the solution corresponding to the central point was injected six times.

The standard solutions of thiocolchicoside within the interval of linearity (0.00696-0.02088 mg/mLs) were prepared in phosphate buffer at pH 7 (0.025M)-acetonitrile-methanol (40:30:30 v/v/vs) and then injected in triplicate; the solution corresponding to the central point has been injected six times.

The tests have been performed for the formulations mentioned in the example 2 and for the commercial formulations Brufen® cream and Muscoril® cream.

Brufen® cream is a formulation containing 10% of ibuprofen with the following excipients: purified water, carboxypolymethylene, phenoxyethanol, polyethylene glycol 300, triethanolamine.

Muscoril® cream is a formulation containing 0.25% of thiocolchicoside with the following excipients: polioxyethilene-20-sorbitan monoleate, sodium lauryl sulfate, spermaceti, mixture of fat alcohols and hydrocarbons, hydrogenated lanolin, stearic acid, sodium alginate, methyl p-hidroxy benzoate, ethyl p-hidroxybenzoate, propyl p-hidroxybenzoate, lavender essence, purified water.

The values found for thiocolchicoside relatively to Muscoril® cream are reported in table 2.

Table 2

| Time (h) | % ($M_t/M_o$) |
|---|---|
| 0 | 0.00 |
| 1 | 0.00 |
| 2 | 1.72 |
| 3 | 4.90 |
| 4 | 8.70 |
| 5 | 14.00 |
| 6 | 17.00 |

The experimental values for ibuprofen relatively to the Brufen® cream are reported in table 3.

Table 3

| Time (h) | % ($M_t/M_o$) |
|---|---|
| 0 | 0.00 |
| 1 | 0.10 |
| 2 | 0.42 |
| 3 | 1.13 |
| 4 | 1.90 |
| 5 | 2.70 |
| 6 | 3.20 |

The experimental values for thiocolchicoside in the formulation 1 are reported in table 4.

Table 4

| Time (h) | % ($M_t/M_o$) |
|---|---|
| 0 | 0.00 |

(continued)

| Time (h) | % ($M_t/M_o$) |
|---|---|
| 1 | 0.00 |
| 2 | 3.51 |
| 3 | 10.02 |
| 4 | 15.65 |
| 5 | 21.55 |
| 6 | 25.67 |

The in experimental values for ibuprofen in formulation 1 are reported in table 5.

Table 5

| Time (h) | % ($M_t/M_o$) |
|---|---|
| 0 | 0.00 |
| 1 | 0.26 |
| 2 | 0.63 |
| 3 | 1.26 |
| 4 | 1.90 |
| 5 | 2.70 |
| 6 | 3.40 |

In tables from 2 to 5 $M_t$ means the amount of drug lost by the compartment 2 and received by the compartment 3; $M_0$ is the amount of drug initially present in the compartment 2; the ratio $M_t/M_0$ is reported as %.

The permeation amounts for thiocolchicoside for formulations 1-15 are reported in table 6.

Table 6

| Formulation | S(cm$^2$) | b(cm) | $t_L$(h) | D(cm$^2$/h) | J(mg/cm$^2$) | Cc (mg/mg) |
|---|---|---|---|---|---|---|
| **1** | 10.70 | 0.180 | 1.46 | 0.0037 | 3.043 | 148.00 |
| **2** | - | 0.198 | 1.00 | 0.0065 | 2.125 | 64.75 |
| **5** | - | 0.207 | 1.45 | 0.0050 | 2.294 | 94.97 |
| **14** | - | 0.206 | 1.48 | 0.0047 | 2.861 | 125.40 |
| **3** | - | 0.191 | 0.82 | 0.0074 | 1.919 | 49.54 |
| **15** | - | 0.168 | 1.00 | 0.0047 | 4.036 | 144.26 |
| **6** | - | 0.191 | 0.63 | 0.0097 | 3.419 | 67.32 |
| **7** | - | 0.213 | 2.15 | 0.0035 | 3.625 | 219.76 |
| **4** | - | 0.202 | 1.36 | 0.0050 | 4.014 | 162.15 |
| **8** | - | 0.167 | 1.78 | 0.0026 | 5.334 | 340.60 |
| **9** | - | 0.147 | 1.49 | 0.0024 | 1.676 | 102.66 |
| **10** | - | 0.185 | 2.73 | 0.0020 | 2.019 | 186.73 |
| **11** | - | 0.171 | 0.91 | 0.0054 | 3.440 | 109.25 |
| **12** | - | 0.167 | 1.45 | 0.0032 | 4.345 | 226.75 |
| **13** | - | 0.205 | 2.23 | 0.0031 | 2.580 | 170.61 |

The in permeation amounts for ibuprofen in formulations 1-15 are reported in table 7.

Table 7

| Formulation | S($cm^2$) | b(cm) | $t_L$(h) | D($cm^2$/h) | J(mg/$cm^2$h) | Cc(mg/mg) |
|---|---|---|---|---|---|---|
| **1** | 10.70 | 0.180 | 1.00 | 0.005 | 588.79 | 19626 |
| **2** | - | 0.198 | 0.87 | 0.007 | 526.19 | 13891 |
| **5** | - | 0.207 | 1.00 | 0.007 | 539.14 | 15718 |
| **14** | - | 0.206 | 1.10 | 0.006 | 643.72 | 20720 |
| **3** | - | 0.191 | 0.96 | 0.006 | 463.68 | 14058 |
| **15** | - | 0.168 | 1.02 | 0.004 | 600.70 | 21938 |
| **6** | - | 0.191 | 1.44 | 0.004 | 1033.90 | 47018 |
| **7** | - | 0.213 | 1.24 | 0.006 | 539.74 | 18908 |
| **4** | - | 0.202 | 1.95 | 0.003 | 1328.60 | 76679 |
| **8** | - | 0.167 | 1.30 | 0.003 | 412.28 | 19554 |
| **9** | - | 0.185 | 1.21 | 0.005 | 1159.10 | 45624 |
| **10** | - | 0.147 | 0.72 | 0.005 | 2475.4 | 72776 |
| **11** | - | 0.201 | 0.73 | 0.007 | 2212.20 | 57216 |
| **12** | - | 0.167 | 0.80 | 0.006 | 2092.30 | 58236 |
| **13** | - | 0.205 | 1.25 | 0,006 | 2000.30 | 67677 |

In tables 6 and 7, S means the surface of the membrane 5; b means the thickness of the membrane 5; $t_L$ is the lag time or the time necessary to permeate the horny layer (in the specific case of the membrane 5); D is the diffusion coefficient and it is calculated with the following equation 1:

$$D = b^2/6t_L \ (cm^2/hours) \ (1);$$

J is the flow is calculated with the following equation (2):

$$J = dM/dt \ S \ (mg/cm^2 \ hours) \ (2);$$

Cc is the concentration of the molecule (thiocolchicoside or ibuprofen) in the horny layer, i.e. the ratio among the weight of the molecule (thiocolchicoside or ibuprofen) absorbed in the horny layer and the weight of the same horny layer; Cc is expressed as % and is calculated by means of the following equation 3:

$$Cc = Jb/D \ (3).$$

From table 6 can be inferred that the introduction of promoters of absorption (enhancers), such as urea or oleic acid, has resulted in an increase of the diffusion coefficient (D), increasing therefore,the ability of the drug to spread in the horny layer.

Passing from the formulation containing paraffin oil to that with Mygliol 812 is observed an increase of the flow (J), as a result of the increase of D.

The emulgels with Escine show good values of the permeation parameters, probably because such substance, having the properties of a tenside determines a good solubilization of thiocolchicoside in the horny layer.

From table 7 it is evident that the flow (J) it increases passing from paraffin containing emulgel, to that containing isopropyl miristate and to that containing Mygliol 812. This is due to the increase of the concentration of the active principle in the vehicle (Cv), as a consequence of the improved solubility in these oils.

The flow also increases with the addition to the formulation of amines, such as DEA and Dmea, as a consequence

of an increase of concentration of ibuprofen in the vehicle (Cv), due to the salification of ibuprofen that can also be dissolved in the aqueous phase in the dissociated form, increasing the partition coefficient vehicle/skin. Moreover, the experimental data also underline an increase of the drug concentration in the horny layer (Cc) that contributes to increase the flow.

It should be pointed out that such parameters can also be influenced by the viscosity of the different emulgels: going from the gel with Mygliol to the gels with Mygliol and Dea/Dmea the viscosity drastically decreases favouring the passage of the active principle from the vehicle to the skin.

The emulgel containing Mygliol, Escine, Dmea and 10% of Sepigel, i.e. the emulgel with a viscosity comparable to that of the emulgels containing Mygliol and without amines, produces an improvement of the permeation parameters however, pointing out that it is more determinant the effect of the salification of ibuprofen that the decrease of the viscosity of the vehicle. This data is also confirmed by the comparison of the permeation of thiocolchicoside with that of ibuprofen in the same emulgels: in the low viscous gels or in the gels in which the amine is present, an significant increase of the absorption is found only for ibuprofen, suggesting that this happens only due to the salification.

**Example 4**

This example describes tests in vivo devised to verify the permeation of thiocolchicoside and ibuprofen into horny layer.

The *in vivo* tests of permeation were performed on healthy volunteers with an age between 23 and 30 years. Before effecting every type of application the volunteers have been acclimatized at a constant temperature and relative humidity. The tests have been carried out at temperatures in the range 20-25 °C and at a relative humidity in the range 55-65%.

The more proper anatomical site were carry on permeation studies is the forearm. Before the application the forearm has been cleaned with 96% ethanol and then with water. The zone of application had a surface 2 x 5 cm or 3 x 3 cm. The sample was applied on the suitable zone in an exactly weighed amount. After an application of 30-60 min, the substance in excess was eliminated with paper and the zone was cleaned with water.

The horny layer of the treated area has been drawn out through the application of bandaids CORNEOFIX® (Courage & Khazaka, Cologne, Germany) applied on the surface of the skin and stuck to it with a force of approximately 1 kg.

The same area has to be drawn out for more times with the bandaids CORNEOFIX®. The bandaids of every voluntary and for every application were collected into a becker containing a suitable amount of solvent and extracted for 1 to 3 hours. Subsequently a quantitative analysis has been carried out for determining the release of the substance from the formulation and its ability to permeate the skin.

The results of the tests for the formulations 9 and 10 are reported in tables 8 and 9, respectively.

Table 8

| Sample | arm | time | pH | hydration | μg/cm$^2$ (thio) | μg/cm$^2$ (ibu) |
|---|---|---|---|---|---|---|
| **1** | sn | 0 mins | 4.9 | 36 | - | - |
| | | 30 mins | 6.5 | 47 | - | 7.80 |
| | dx | 0 mins | 4.8 | 36 | - | - |
| | | 6 hs | 6.4 | 34 | 0.66 | 102.60 |
| **2** | sn | 0 mins | 4.6 | 39 | - | - |
| | | 30 mins | 6.5 | 50 | - | 7.80 |
| | dx | 0 mins | 4.9 | 40 | - | |
| | | 6 hs | 6.4 | 40 | 0.74 | 175.20 |
| **3** | sn | 0 mins | 5.1 | 28 | - | - |
| | | 30 mins | 6.3 | 34 | - | 7.20 |
| | dx | 0 mins | 5.3 | 27 | - | - |
| | | 6 hs | 6.4 | 28 | 0.71 | 177.00 |
| **4** | sn | 0 mins | 5.6 | 38 | - | - |
| | | 30 mins | 6.3 | 39 | - | 7.20 |

(continued)

| Sample | arm | time | pH | hydration | µg/cm² (thio) | µg/cm² (ibu) |
|---|---|---|---|---|---|---|
| | dx | 0 mins | 5.1 | 35 | - | - |
| | | 6 hs | 6.1 | 32 | 0.73 | 174.00 |
| **5** | sn | 0 mins | 5.0 | 34 | - | - |
| | | 30 mins | 6.3 | 40 | - | 7.80 |
| | dx | 0 mins | 5.2 | 35 | - | - |
| | | 6 hs | 6.2 | 34 | 0.46 | 103.20 |
| **6** | sn | 0 mins | 4.8 | 39 | - | - |
| | | 30 mins | 6.5 | 43 | - | 7.20 |
| | dx | 0 mins | 5.1 | 37 | - | - |
| | | 6 hs | 6.3 | 35 | 0.50 | 103.20 |

Table 9

| Sample | arm | time | pH | hydration | µg/cm² (thio) | µg/cm² (ibu) |
|---|---|---|---|---|---|---|
| **1** | sn | 0 mins | 5.2 | 31 | - | - |
| | | 30 mins | 6.6 | 45 | - | 9.00 |
| | dx | 0 mins | | 35 | - | - |
| | | 6 hs | 6.3 | 39 | 0.66 | 106.20 |
| **2** | sn | 0 mins | 4.6 | 39 | - | - |
| | | 30 mins | 6.8 | 43 | - | 12.60 |
| | dx | 0 mins | 4.9 | 42 | - | - |
| | | 6 hs | 6.5 | 30 | 0.94 | 181.00 |
| **3** | sn | 0 mins | 4.8 | 31 | - | - |
| | | 30 mins | 6.6 | 32 | - | 12.00 |
| | dx | 0 mins | 5.1 | 33 | - | - |
| | | 6 hs | 6.4 | 20 | 0.74 | 179.20 |
| **4** | sn | 0 mins | 5.2 | 32 | - | - |
| | | 30 mins | 6.4 | 39 | - | 11.40 |
| | dx | 0 mins | 4.8 | 36 | - | - |
| | | 6 hs | 5.8 | 29 | 0.97 | 175.00 |
| **5** | sn | 0 mins | 5.0 | 41 | - | - |
| | | 30 mins | 6.3 | 43 | - | 9.00 |
| | dx | 0 mins | 5.2 | 39 | - | - |
| | | 6 hs | 6.2 | 30 | 0.85 | 182.40 |
| **6** | sn | 0 mins | 4.9 | 34 | - | - |
| | | 30 mins | 6.4 | 40 | - | 8.00 |
| | dx | 0 mins | 5.1 | 36 | - | - |
| | | 6 hs | 6.2 | 30 | 0.65 | 104.40 |

In the tables 8 and 9 thio means thiocolchicoside and ibu means ibuprofen.

For both the formulations 9 and 10, subsequently to application a light increase of the hydration is noticed (Corneometer CM 820, Courage & Khazaka, Cologne. Germany) particularly after 30 min; hydration after 6 hours seems to return to the initial value pointing out the non occlusive nature of the vehicle.

For both formulations 9 and 10, an increase of the pH is observed (Skin-PH-meter PH 900, Courage & Khazaka, Cologne. Germany) which can be related to the presence of basic substances in the vehicle (DEA or Dmea); such pH variation also suggests the possibility to transfer to the horny layer the antiinflammatory in the dissociated form improving its solubility (Cc) as it is also verified by the *in vitro* tests.

For both the formulations 9 and 10, no absorption of thiocolchicoside in the horny layer is detected after 30 min; this is in accord with the lag times calculated in the in vitro tests that always exceed such time.

Formulation 10 containing the Dmea has involved a greater transfer in the skin of the two active principles; such situation is not referable to variations of pH or hydration but rather to an increase of the permeation parameters, as previously already observed by the evaluation of the tests in vitro.

## Example 5

The present example describes tests carried out *in vivo* that confirm the surprising anti-inflammatory and/or analgesic activity of the formulations in accordance with the present invention.

<u>Case 1</u>

Subject: female of age exceeding 50 years. Post-operating painful and inflammatory syndrome (removal of malleolar screw and plate following fracture of both malleoli). Treatment with the formulation 12 for 30 days (with 3 daily applications). Two applications morning and evening and an application before the reabilitative therapy.

Clinical results: decrease of the muscular rigidity, attenuation of the pain and improvement of the general clinical parameters already after the first week of pharmacological therapy.

<u>Case 2</u>

Subject: male of age exceeding 50 years.

Post-traumatic painful syndrome following the fracture of the radial capital (elbow). After the second week of rehabilitation following the removal of the chalked bandages, treatment (with 3 daily applications) with the formulation 1 for fifteen days.

Result: Clinical recovery.

<u>Case 3</u>

Subject: female of age exceeding 80 years.

Painful syndrome to a knee struck by chronic rheumatoide arthritis.

Clinical results: improvement of the symptomatology with recovery of the nighttime sleep thanks of the decrease of the pain at the level of the inflammed tissues after a week of applications (three daily applications).

<u>Case 4</u>

Subject: female of age exceeding 30 years.

Acute post-traumatic syndrome following an acute lumbar sciatica.

Treatment with the formulation 12 up to complete remission of the painful symptomatology (ten days).

## Claims

1. Formulation for transdermal use comprising an emulgel, thiocolchicoside and a substantially lipohilic compound, having a pharmacological activity, which is selected from the group consisting of: ibuprofen, ketoprofen, naproxen and sulindac.

2. Formulation according to claim 1, wherein the substantially lipophilic compound is ibuprofen.

3. Formulation according to Claim 2, wherein the concentration of ibuprofen is between 2%wt and 16%wt.

4. Formulation according to one of the foregoing claims, wherein the concentration of thiocolchicoside is between 0.03% and 0.25%.

5. Formulation according to one of the foregoing claims, wherein the emulgel comprises water and/or propylene glycol.

6. Formulation according to claim 5, wherein the w/w % of water is between 44% and 65%.

7. Formulation according to one of the foregoing claims, wherein the emulgel comprises polyacrylamide and C13-C14 isoparaffin and Laureth-7.

8. Formulation according to one of the foregoing claims, wherein the emulgel comprises Sepigel 305.

9. Formulation according to claim 8, wherein the w/w % of Sepigel 305 is in the range of 2% and 11%.

10. Formulation according to one of the foregoing claims, wherein the emulgel comprises water and/or propylene glycol; and the w/w % of propylene glycol is of 5% with respect to the formulation.

11. Formulation according to one of the foregoing claims, wherein the emulgel comprises Mygliol 812.

12. Formulation according to one of the foregoing claims, wherein the emulgel comprises Cremophor RH40.

13. Formulation according to one of the foregoing claims, wherein the emulgel comprises an oil or a mixture of oils, in which the lipophilic compound has a solubility between 5% and 20%.

14. Formulation according to claim 13, wherein the solubility of the lipophilic compound is between 8.2% and 14.5%.

15. Formulation for transdermal use comprising thiocolchicoside, ibuprofen and a pharmaceutically acceptable vehicle suitable for transdermal use.

16. Pharmaceutical formulation according to claim 15, wherein the suitable pharmaceutical vehicle comprises an emulgel.

17. Formulation according to claim 16, wherein the emulgel is defined in accordance with one of claims 5 to 14.

18. Formulation according to one of the foregoing claims wherein said formulation comprises at least a promoter of absorption.

19. Formulation according to one of claims 15 to 18, wherein the concentration of thiocolchicoside is between 0.03% and 0.25%.

20. Formulation according to one of the foregoing, claims, wherein the concentration of ibuprofen is between 4% and 12%.

21. Formulation according to one of the foregoing claims for use as a pharmaceutical.

22. Formulation according to one of the foregoing claims for use in the treatment of pain and/or inflammation.

23. Formulation for use according to claim 21 or 22, wherein the formulation is administered topically.

24. Use of a formulation according to one of the foregoing claims, for the preparation of a pharmaceutical product for the therapy of pain and/or inflammation in mammals.

25. Formulation for use according to claim 24, wherein the formulation is administered topically.

## Patentansprüche

1. Rezeptur zur transdermalen Anwendung, die ein Emulgel, Thiocolchicosid und eine im Wesentlichen lipophile Verbindung aufweist, die eine pharmakologische Aktivität hat und aus der Gruppe ausgewählt ist, die aus folgendem

besteht: Ibuprofen, Ketoprofen, Naproxen und Sulindac.

**2.** Rezeptur nach Anspruch 1, wobei die im Wesentlichen lipophile Verbindung Ibuprofen ist.

**3.** Rezeptur nach Anspruch 2, wobei die Konzentration des Ibuprofen zwischen 2 Gew.-% und 16 Gew.-% ist.

**4.** Rezeptur nach einem der vorhergehenden Ansprüche, wobei die Konzentration des Thiocolchicosides zwischen 0,03 % und 0,25 % ist.

**5.** Rezeptur nach einem der vorhergehenden Ansprüche, wobei das Emulgel Wasser und/oder Propylenglycol aufweist.

**6.** Rezeptur nach Anspruch 5, wobei das w/w % des Wassers zwischen 44% und 65% ist.

**7.** Rezeptur nach einem der vorhergehenden Ansprüche, wobei das Emulgel Polyacrylamid und C13-C14 Isoparaffin und Laureth-7 aufweist.

**8.** Rezeptur nach einem der vorhergehenden Ansprüche, wobei das Emulgel Sepigel 305 aufweist.

**9.** Rezeptur nach Anspruch 8, wobei das w/w % des Sepigel 305 im Bereich von 2% bis 11 % ist.

**10.** Rezeptur nach einem der vorhergehenden Ansprüche, wobei das Emulgel Wasser und/oder Propylenglycol aufweist; und wobei das w/w % des Propylenglycol 5% der Rezeptur ausmacht.

**11.** Rezeptur nach einem der vorhergehenden Ansprüche, wobei das Emulgel Mygliol 812 aufweist.

**12.** Rezeptur nach einem der vorhergehenden Ansprüche, wobei das Emulgel Cremophor RH40 aufweist.

**13.** Rezeptur nach einem der vorhergehenden Ansprüche, wobei das Emulgel ein Öl oder eine Mischung von Ölen aufweist, wobei die lipophile Verbindung eine Lösbarkeit zwischen 5% und 20% hat.

**14.** Rezeptur nach Anspruch 13, wobei die Lösbarkeit der lipophilen Verbindung zwischen 8,2 % und 14,5 % ist.

**15.** Rezeptur zur transdermalen Anwendung, welche Thiocolchicosid, Ibuprofen und ein pharmazeutisch akzeptables Transportmittel aufweist, welches zur transdermalen Anwendung geeignet ist.

**16.** Pharmazeutische Rezeptur nach Anspruch 15, wobei das geeignete pharmazeutische Transportmittel ein Emulgel aufweist.

**17.** Rezeptur nach Anspruch 16, wobei das Emulgel gemäß einem der Ansprüche 5 bis 14 definiert ist.

**18.** Rezeptur nach einem der vorhergehenden Ansprüche, wobei die Rezeptur zumindest einen Promotor für die Absorption aufweist.

**19.** Rezeptur nach einem der Ansprüche 15 bis 18, wobei die Konzentration des Thiocolchicosides zwischen 0,03 % und 0,25 % ist.

**20.** Rezeptur nach einem der vorhergehenden Ansprüche, wobei die Konzentration des Ibuprofens zwischen 4 % und 12 % ist.

**21.** Rezeptur nach einem der vorhergehenden Ansprüche zur Verwendung als Pharmazeutikum.

**22.** Rezeptur nach einem der vorhergehenden Ansprüche zur Verwendung bei der Behandlung von Schmerzen und/oder Entzündungen.

**23.** Rezeptur zur Verwendung nach Anspruch 21 oder 22, wobei die Rezeptur äußerlich verabreicht wird.

**24.** Verwendung einer Rezeptur nach einem der vorhergehenden Ansprüche zur Zubereitung eines pharmazeutischen Produktes zur Behandlung von Schmerzen und/oder Entzündungen bei Säugetieren.

**25.** Rezeptur zur Anwendung nach Anspruch 24, wobei die Rezeptur äußerlich angewendet wird.

**Revendications**

**1.** Formulation pour une utilisation transdermique, comprenant un émulgel, un thiocolchicoside et un composé essentiellement lipophile, ayant une activité pharmacologique, qui est choisi dans le groupe constitué par : l'ibuprofène, le kétoprofène, le naproxène et le sulindac.

**2.** Formulation selon la revendication 1, dans laquelle le composé essentiellement lipophile est l'ibuprofène.

**3.** Formulation selon la revendication 2, dans laquelle la concentration d'ibuprofène est entre 2 % en poids et 16 % en poids.

**4.** Formulation selon l'une des revendications précédentes, dans laquelle la concentration de thiocolchicoside est entre 0,03 % et 0,25 %.

**5.** Formulation selon l'une des revendications précédentes, dans laquelle l'émulgel comprend de l'eau et/ou du propylèneglycol.

**6.** Formulation selon la revendication 5, dans laquelle le % en p/p d'eau est entre 44 % et 65 %.

**7.** Formulation selon l'une des revendications précédentes, dans laquelle l'émulgel comprend un polyacrylamide et une isoparaffine en C13-C14 et du Laureth-7.

**8.** Formulation selon l'une des revendications précédentes, dans laquelle l'émulgel comprend du Sépigel 305.

**9.** Formulation selon la revendication 8, dans laquelle le % en p/p de Sépigel 305 est dans la gamme de 2 % à 11 %.

**10.** Formulation selon l'une des revendications précédentes, dans laquelle l'émulgel comprend de l'eau et/ou du propylèneglycol ; et le % en p/p du propylène-glycol est de 5 % par rapport à la formulation.

**11.** Formulation selon l'une des revendications précédentes, dans laquelle l'émulgel comprend du Mygliol 812.

**12.** Formulation selon l'une des revendications précédentes, dans laquelle l'émulgel comprend du Crémophor RH40.

**13.** Formulation selon l'une des revendications précédentes, dans laquelle l'émulgel comprend une huile ou un mélange d'huiles, dans lesquelles le composé lipophile a une solubilité entre 5 % et 20 %.

**14.** Formulation selon la revendication 13, dans laquelle la solubilité du composé lipophile est entre 8,2 % et 14,5 %.

**15.** Formulation pour une utilisation transdermique, comprenant du thiocolchicoside, de l'ibuprofène et un véhicule pharmaceutiquement acceptable approprié pour une utilisation transdermique.

**16.** Formulation pharmaceutique selon la revendication 15, dans laquelle le véhicule pharmaceutique approprié comprend un émulgel.

**17.** Formulation selon la revendication 16, dans laquelle l'émulgel est défini selon l'une des revendications 5 à 14.

**18.** Formulation selon l'une des revendications précédentes, ladite formulation comprenant au moins un promoteur d'absorption.

**19.** Formulation selon l'une des revendications 15 à 18, dans laquelle la concentration de thiocolchicoside est entre 0,03 % et 0,25 %.

**20.** Formulation selon l'une des revendications précédentes, dans laquelle la concentration d'ibuprofène est entre 4 % et 12 %.

**21.** Formulation selon l'une des revendications précédentes, pour une utilisation en tant que produit pharmaceutique.

**22.** Formulation selon l'une des revendications précédentes, pour une utilisation dans le traitement d'une douleur et/ou d'une inflammation.

**23.** Formulation pour une utilisation selon la revendication 21 ou 22, laquelle formulation est administrée localement.

**24.** Utilisation d'une formulation selon l'une des revendications précédentes, pour la préparation d'un produit pharmaceutique pour la thérapie d'une douleur et/ou d'une inflammation chez des mammifères.

**25.** Formulation pour une utilisation selon la revendication 24, laquelle formulation est administrée localement.

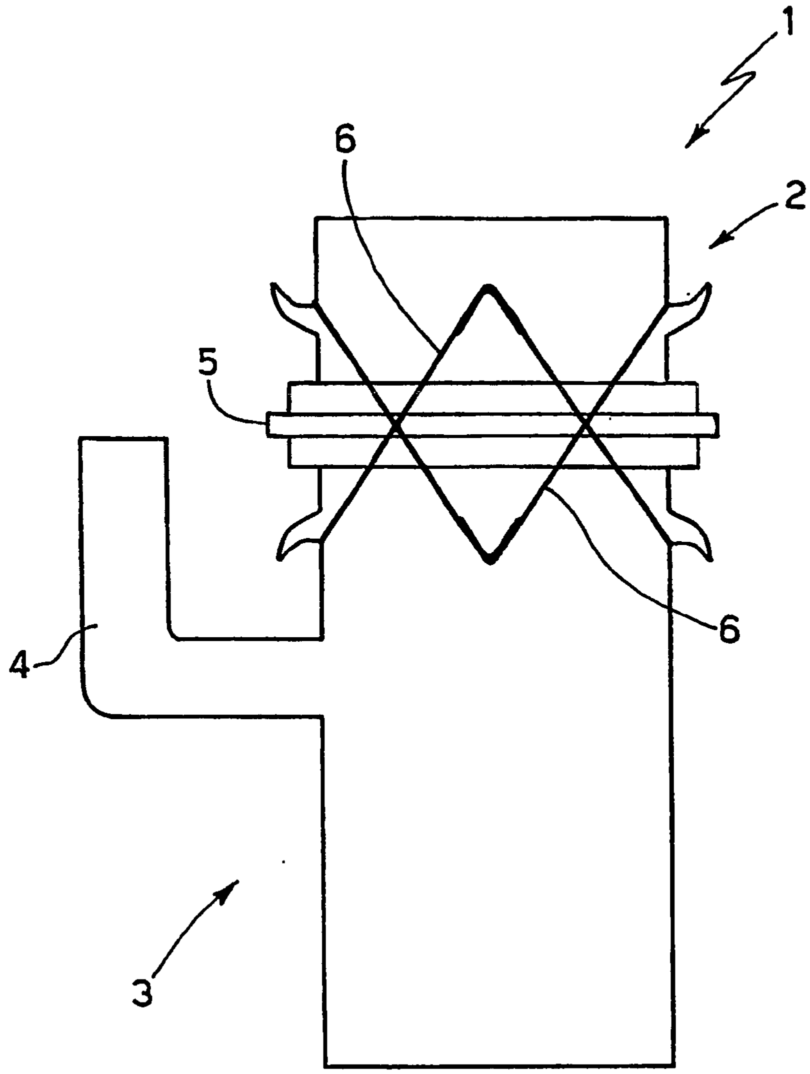


Fig.1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6399093 B
- WO 9641635 A
- FR 2725134

### Non-patent literature cited in the description


- **PIFFERI G.** COMPATIBILITA CHIMICO-FI SICA TRA TIOCOLCHICOSIDE E FARMACI ANTINFIAMMATORI NON STEROIDEI. - Chemical-physical compatibility of thiocolchicoside with nonsteroidic anti-inflammatory drugs. *BOLLETTINO CHIMICO FARMACEUTICO, SOCIETA EDITORIALE FARMACEUTICA, MILANO, IT,* 01 June 1993, vol. 132 (6), 203-209
- **LA ROTONDA, M. I. ; AMATO, G. ; BARBATO, F. ; SILIPO, C. ; VITTORIA, A.** *Quant. Struct. Act., Relat.,* 1983, vol. 2, 168